# EUROPEAN PATENT APPLICATION

(11) **EP 0 522 737 A1**
(43) Date of publication of application: **13.01.1993**
(21) Application number: 92305741.8
(22) Date of filing: 23.06.1992
(51) Int. Cl.: A61M 25/06, A61B 17/34

(54) **Clear cannula hub**

(30) Priority: 08.07.1991 US 726452
(71) Applicant: BAXTER INTERNATIONAL INC., Deerfield, IL 60015 (US)
(72) Inventor: Schaechter, Ronald, Los Angeles, California 90035 (US)
(74) Representative: MacGregor, Gordon

(57) **Abstract**

A hub is described for use with spinal needles. The hub includes a convex lens which acts as a magnifier of any fluid in the hub. The lens allow medical personnel to more easily see any fluid which may be present in the hub.

## Description

### FIELD OF THE INVENTION

This invention relates generally to hubs for attachment to cannulas and more specifically relates to hubs for attachment to cannulas for draining cerebral-spinal fluid from a patient or injected fluids into a patient.

### BACKGROUND OF THE INVENTION

Various transparent hubs have been used in the past for attachment to cannulas that either drain or infuse fluids into a patient's body. One advantage of using a transparent hub is that it is possible to visually detect any fluid that may be present in the hub. It is particularly important to be able to view fluid in a hub when a cannula is initially inserted in a patient's body. For instance, when an intravenous cannula is initially inserted, it is desirable to be able to determine the presence or absence of blood in the hub. If an intravenous cannula has been correctly inserted into a vein, blood will back up through the cannula to the hub. Thus, the presence of blood in the hub indicates that the cannula has been correctly inserted. On the other hand, the absence of blood indicates that the cannula may not have been correctly inserted into a vein.

In a similar manner, when a spinal cannula is inserted, it is desirable to be able to view the presence or absence of blood or other fluids that may back up through the cannula to assist in determining the appropriate location of the cannula. In many instances, the cannula and hub may be relatively small, and the amount of fluid in the hub may be comparatively minute. In such instances, it may be difficult to see any fluid that may be present in the hub. Therefore, a need existed to develop a hub which includes means for visualizing fluid therein.

### SUMMARY OF THE INVENTION

A device for use in transporting fluids between a container and a patient is described. The device includes a housing that has upper and lower walls. The housing also has first and second generally opposing end walls that connect the upper and lower walls. The housing also includes a bore between the upper and lower walls that extends from the first to the second end wall. An interface between the first end wall and the bore defines a first port, and an interface between the second end wall and the bore defines a second port. The bore is adapted to transport fluids between the ports.

The device also includes a cannula for insertion into a patient. The cannula is connected to the first port.

The device also includes a means for visually magnifying a portion of any fluid in the bore. In the preferred embodiment of the invention, the means for visually magnifying includes a transparent lens portion extending between the bore and the upper wall, the lens portion forming a convex surface extending outwardly from the upper surface.

### BRIEF DESCRIPTION OF THE DRAWINGS

**Fig. 1** is a perspective view of the preferred embodiment of the invention;
**Fig. 2** is a side view of the preferred embodiment of the invention;
**Fig. 3** is a top view of the preferred embodiment of the invention; and
**Fig. 4** is a perspective cross-sectional view of the preferred embodiment of the invention.

### DETAILED DESCRIPTION OF THE PREFERRED EMBODIMENT

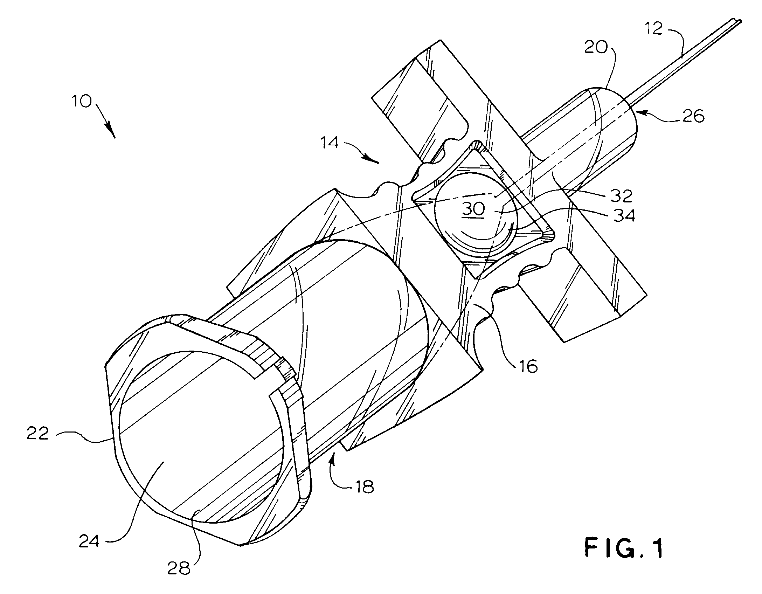

Refer now to **Fig. 1** which is a perspective view of the preferred embodiment of the invention. As can be seen in the figure, the invention is a device **10** for use in transporting fluids to and from a patient. The device **10** includes a cannula **12** for insertion into a patient. The cannula **12** is a hollow tube which may be inserted into either the vein of a patient or into a patient's spinal area. The device **10** also includes a housing **14**. The housing **14** includes upper and lower walls **16** and **18**, respectively. The housing also includes first and second end walls **20** and **22**, respectively, which connect the upper and lower walls. The first and second end walls in the preferred embodiment are generally parallel with one another. In the preferred embodiment, the upper and lower walls are also generally parallel. However, in other embodiments, the walls may take other shapes.

The housing **14** also includes a bore **24** that is located between the upper and lower walls **16, 18**. The bore **24** extends from the first to the second end wall. An interface between the bore and the first end wall defines a first port **26**. Similarly, an interface between the second end wall **22** and the bore **24** forms a second port **28**. The first port **26** is in fluid connection with one end of the cannula **12**. The bore is adapted to transport fluids between the first and second ports **26**, **28**. Therefore, any fluid from a patient that is transported through the cannula **12** travels through the housing from the first end wall **20** to the second end wall **22** through the bore **24**. In order to more easily see any such fluid, the subject invention includes a means **30** for visually magnifying a portion of any fluid in the bore.

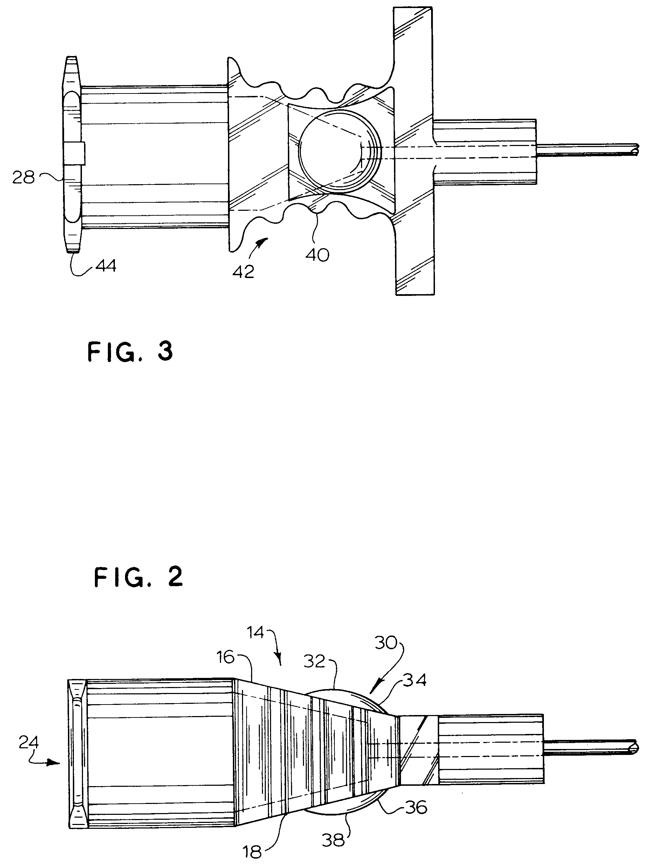

In the preferred embodiment of the invention, the means **30** includes a transparent lens portion **32** which extends between the bore and the upper wall. The lens portion forms a convex surface **34** which extends outwardly from the upper wall. The convex surface **34** can be more clearly seen in **Fig. 2,** which is a side view of the preferred embodiment of the invention. As also can be seen in **Fig. 2,** the means **30** for visually magnifying a portion of any fluid in the bore **24** includes a second lens **36** that extends from the lower wall **18** of the housing **14**. The second lens **36** also includes a convex surface **38**. Both the first and second lens are identical in that they are transparent and extend between the bore **24** to the upper and lower walls **16**, **18**, respectively. These lenses serve to magnify any fluid present in the bore **24**.

Refer now to **Fig. 3.** As can be seen in the figure, in the preferred embodiment of the invention, the housing includes a gripping means for grasping the housing during insertion or removal of a cannula from a patient. In the preferred embodiment, the gripping means **40** includes a plurality of ridged surfaces **42** which are generally parallel to one another and extend from the upper wall **16** to the lower wall **18** between the first and second end walls **20**, **22**. In the preferred embodiment, the housing also includes a luer lock **44** at the second port **28**. The luer lock allows the device to be attached to a syringe, medication tubing or other fluid container.

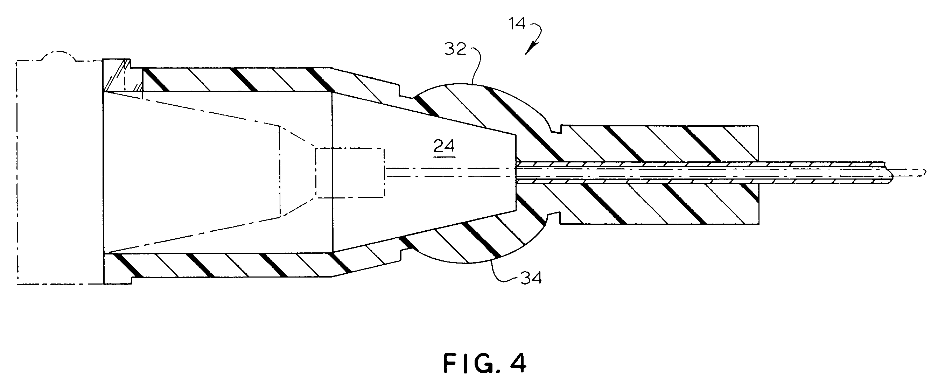

Refer now to **Fig. 4**, which is a perspective cross-sectional view. As can be seen in the figure, the increased thickness of the housing **14** at the lens **32** and the convex surface **34** of the lens allows fluid in the bore **24** to be magnified.

While the invention has been described in detail and with reference to specific embodiments thereof, it will be apparent to those skilled in the art that various changes and modifications can be made therein without departing from the spirit and scope of the invention.

## Claims

1. A device **(10)** for use in transporting fluids to and from a patient, comprising:
a cannula **(12)** for insertion into a patient,
a housing **(14)**, said housing having
upper and lower walls **(16)**, **(18)**;
first and second end walls **(20)**, **(22)** connecting said upper and lower walls;
a bore **(24)** between said upper and lower walls extending from said first to said second end wall to define a first port at an interface between said first end wall and said bore and a second port at an interface between said second end wall and said bore, said first port being connected to said cannula, said bore being adapted to transport fluids between said ports; and
means **(30)** for visually magnifying a portion of any fluid in said bore.

2. A device as recited in Claim **1**, wherein said means **(30)** for visually magnifying further includes:
a transparent lens portion **(32)** extending between said bore **(24)** and said upper wall **(16)**, said lens portion forming a convex surface **(34)** extending outwardly from said upper wall.

3. A device as recited in Claim **3**, wherein said means **(30)** for visually magnifying and said housing **(14)** are integrally formed from a single piece of transparent plastic.

4. A device as recited in Claim **1**, wherein said means for visually magnifying further includes:
first and second transparent lens portions, said first transparent lens portion extending between said bore and said upper wall, and said second transparent lens portion extending between said bore and said lower wall, said lens portions forming first and second convex surfaces which extend outwardly from said upper and lower surfaces, respectively.
